# EUROPEAN PATENT APPLICATION

(11) **EP 4 134 090 A1**
(43) Date of publication of application: **15.02.2023**
(21) Application number: 21785700.2
(22) Date of filing: 10.04.2021
(51) Int. Cl.: A61K 35/747, A61P 25/16, A61P 25/20, A61K 35/741, A61K 35/742, A61K 35/745

(54) **PROBIOTIC COMPOSITION FOR TREATING RAPID EYE MOVEMENT SLEEP BEHAVIOR DISORDER, FORMULATION AND USE**

(30) Priority: 10.04.2020 CN 202010281549
(71) Applicant: Beijing Friendship Hospital Affiliated To Capital Medical University, Beijing 100050 (CN)
(72) Inventor: TUO, Houzhen, Beijing 100050 (CN); DU, Yitong, Beijing 100050 (CN); XU, Xiaojiao, Beijing 100050 (CN); LI, Yue, Beijing 100050 (CN); ZHANG, Mingkai, Beijing 100050 (CN); CUI, Ying, Beijing 100050 (CN); XUE, Yun, Beijing 100050 (CN); GAO, Dan, Beijing 100050 (CN); GAO, Ting, Beijing 100050 (CN); SHENG, Zhi, Beijing 100050 (CN)
(74) Representative: Wang, Bo
(86) International application number: PCT/CN2021/086312
(87) International publication number: WO 2021/204279

(57) **Abstract**

A probiotic composition for treating rapid eye movement sleep behavior disorder, being prepared from *Bacillus licheniformis, Bifidobacterium longum, Lactobacillus acidophilus, Enterococcus faecalis,* and pharmaceutically acceptable adjuvants. The probiotic composition can improve the symptoms of rapid eye movement sleep behavior disorder of a patient, improve RBD symptoms of idiopathic RBD patients and PD patients, and reduce the dosage of levodopa in PD patients.

## Description

### BACKGROUND

### Technical Field

The present disclosure relates to a probiotic composition, specifically to a probiotic composition for treating rapid eye movement sleep behavior disorder and a formulation obtained from the composition.

### Related Art

Rapid eye movement sleep behavior disorder (RBD for short) refers to a sleep disorder that occurs when a patient enters the rapid eye movement sleep period. Because the muscles cannot be completely relaxed, there are cries, words or physical activities associated with dreams. The most common behavior is to swing your arms on the bed, kick, talk, shout, get up, or grind your teeth, laugh, sing, call, or even fall into bed. These patients generally need to be treated with drugs. Currently effective drugs include clonazepam, melatonin and other drugs, but long-term use of clonazepam may lead to cognitive decline of patients; Moreover, it is easy to produce dependence and drug resistance, with large side effects.

Idiopathic RBD patients may develop into nervous system degenerative diseases after several years, such as Parkinson's Disease (PD), Lewy body dementia, multi system atrophy, etc. RBD is a common non motor symptom of Parkinson's disease, which is seen in more than half of Parkinson's patients. At present, the therapeutic drugs for Parkinson's disease mainly include the following categories: mainly to improve the level of dopaminergic transmitters in the synaptic space of neurons, including levodopa preparations, dopamine receptor agonists, monoamine oxidase inhibitors, COMT inhibitors, etc; Regulate the action of other neurotransmitters such as acetylcholine or NMDA, including benzhexol hydrochloride, amantadine, etc. These drugs can only control exercise symptoms, and cannot delay the progress of the disease. Moreover, with the progress of the disease and long-term drug treatment, the amount and types of drugs used by patients will gradually increase, but the efficacy will decline, and sports complications such as "motion fluctuation", "dyskinesia" and "switching phenomenon" will occur. Moreover, the drugs used to treat Parkinson's disease can effectively improve the motor symptoms of Parkinson's disease, but cannot improve the RBD symptoms at the same time, and some drugs may even aggravate the frequency of RBD attacks.

### SUMMARY

The primary technical problem to be solved by the present disclosure is to provide a probiotic composition for treating rapid eye movement sleep behavior disorder.

Another technical problem to be solved by the present disclosure is to provide a probiotic preparation prepared by using the probiotic composition.

Another technical problem to be solved by the present disclosure is to propose a new use of the above probiotic composition and preparation.

In order to achieve the above purposes, the present disclosure adopts the following technical solutions:
According to the first aspect of the embodiment of the present disclosure, provide a probiotic composition for treating rapid eye movement sleep behavior disorder, is prepared from *Bacillus licheniformis, Bifidobacterium longum, Lactobacillus acidophilus, Enterococcus faecalis* and acceptable adjuvants in the art.

Preferably, the viable count of *Bacillus licheniformis* in the probiotic composition is not less than 10.0×10⁸ CFU/g; the viable count of *Bifidobacterium longum* in the probiotic composition is not less than 4.0×10⁸ CFU/g; the viable count of *Lactobacillus acidophilus* in the probiotic composition is not less than 1.0×10⁸ CFU/g; and the viable count of *Enterococcus faecalis* in the probiotic composition is not less than 0.5×10⁸ CFU/g.

The above preferred range can also be extended to the following range. The inventors conducted clinical trials using the probiotic composition within the following range and confirmed that the following range can also achieve the technical effect described in the present disclosure: the viable count of *Bacillus licheniformis* in the probiotic composition is not less than 1×10⁷ CFU/g; the viable count of *Bifidobacterium longum* in the probiotic composition is not less than 1×10⁷ CFU/g; the viable count of *Lactobacillus acidophilus* in the probiotic composition is not less than 1×10⁷ CFU/g; and the viable count of *Enterococcus faecalis* in the probiotic composition is not less than 1×10⁷ CFU/g.

Preferably, the viable count of bacteria of the probiotic composition can also be controlled within the following range, but not limited to the following range, and each strain can have a good synergistic effect. The viable count of *Bacillus licheniformis* is 10.0×10⁸ ~ 10.0×10¹⁰CFU/g; the viable count of *Bifidobacterium longum* is 4.0×10⁸∼4.0×10¹⁰CFU/g; the viable count of *lactobacillus acidophilus* is 1.0× 10⁸~1.0×10¹⁰CFU/g; the viable count of *enterococcus faecalis* is 0.5×10⁸∼0.5×10¹⁰CFU/g_{∘}

The above probiotic composition has been proved by clinical trials to have a good therapeutic effect on the RBD symptoms of patients with idiopathic RBD and Parkinson's disease, and the efficacy is stable and persistent. At the same time, it also has a good improvement effect on the motor symptoms of patients with Parkinson's disease. It can play a key protective role in the treatment of patients with Parkinson's disease, and effectively reduce the dosage of levodopa in patients with Parkinson's disease.

According to the second aspect of the embodiment of the present disclosure, a probiotic preparation for treating sleep behavior disorder during rapid eye movement sleep period is provided, which is made of the above probiotic composition and acceptable adjuvants in the art. The adjuvants are used to prepare different probiotic dosage forms. The acceptable adjuvants in the art include but are not limited to solvent, emulsifier, disintegrant, solubilizer, antioxidant, pH regulator, osmotic pressure regulator, bacteriostatic agent, bacteriostatic agent Diluent, wetting agent, adhesive, film forming agent, etc.

According to the third aspect of the embodiment of the present disclosure, a preparation method of probiotics for treating sleep behavior disorder during rapid eye movement sleep period is provided, which includes the following steps:
(1) preparing of bacterial powder: respectively prepare the dried bacterial powder of *Bacillus licheniformis, Bifidobacterium longum, Lactobacillus acidophilus* and *Enterococcus faecalis;*
(2) Mixing each dried bacterial powder obtained in step (1) with the adjuvants acceptable in the art to prepare the final dosage form.

The adjuvants described in step (2) above can be selected according to the prepared dosage form, for example, the solid preparation can be starch, dextrin, lactose, microcrystalline cellulose or inorganic salts; Wetting agent and adhesive can be starch slurry, glue slurry, etc; Disintegration agent can be dry starch, sodium carboxymethyl starch, effervescent disintegrant, etc; The film coating can be enteric coated.

The use of the probiotic composition in preparing probiotic preparations for RBD.

The above probiotics are used in the preparation of drugs, health supplements and/or food for the treatment of RBD.

The use of probiotics prepared by the above method in the preparation of drugs, health products and/or food for the treatment of RBD.

According to the fourth aspect of the embodiment of the present disclosure, a food composition or supplement for treating sleep behavior disorder during rapid eye movement sleep period is provided, wherein:
The probiotic composition is prepared from *Bacillus licheniformis, Bifidobacterium longum, Lactobacillus acidophilus, Enterococcus faecalis,* and pharmaceutically acceptable adjuvants, wherein
the viable count of *Bacillus licheniformis* in the probiotic composition is not less than 1×10⁷ CFU/g;
the viable count of *Bifidobacterium longum* in the probiotic composition is not less than 1×10⁷ CFU/g;
the viable count of *Lactobacillus acidophilus* in the probiotic composition is not less than 1×10⁷ CFU/g; and
the viable count of *Enterococcus faecalis* in the probiotic composition is not less than 1×10⁷ CFU/g.

The use of the above food composition or supplement in the preparation of a health product supplement and/or food for the treatment of sleep behavior disorders in the rapid eye movement phase of Parkinson's disease.

The above types of food include but are not limited to: biscuits, dairy products, meal substitutes, meat products, sauces, ice cream, fermented products, fruit juice, rice wine, candy, canned food, salted products, condiments, bean products, chocolate, fillings, tea products, puffed food, etc. The food can also be a food additive.

The types of the above supplements for health products include but are not limited to: medicinal liquor, capsules, tablets, granules, tea products, oral liquid, granules, fermented products, honey cream, dew, powder, meal substitutes, etc. The health care product can also include a health care product additive.

*Bacillus licheniformis, Bifidobacterium longum, Lactobacillus acidophilus* and *Enterococcus faecalis* used in the present disclosure (Enterococcus faecalis) are all well-known strains for those skilled in the art, and the freeze-dried powder of the strains of the present disclosure can be obtained through market purchase. The freeze-dried powder only needs to meet the requirements of the living bacteria of the present disclosure, and can be compounded, mixed, and sub packaged according to the well-known methods in the art. The probiotic composition can also be combined with acceptable adjuvants to prepare products with different needs, such as powders, granules, tablets, capsules, etc., according to the well-known drug, health product and food preparation methods. The probiotic live bacteria used in the present disclosure can also be cultivated by the well-known cultivation method in the art. The preparation method and the compound shaping method of probiotics are not limited to the contents disclosed in the present disclosure, and any well-known conventional method can be used to realize the present disclosure.

The above culture methods include but are not limited to: liquid fermentation, solid fermentation and semi-solid fermentation.

The types of the above liquid fermentation methods include but are not limited to: batch fermentation, continuous fermentation and fed batch fermentation. The medium used for the liquid fermentation method includes but is not limited to LB medium, AAM-AS liquid medium, MRS medium, PTYG medium, PY medium, YPD medium and BSM medium; And its improved, deformed and optimized types.

The types of the above solid fermentation methods include but are not limited to: natural enrichment solid fermentation, enhanced microbial mixed solid fermentation, limited microbial mixed solid fermentation, and single strain solid state pure breed fermentation. The culture medium used for the solid fermentation method includes but is not limited to LB solid culture medium, YEB solid culture medium, Sabolol agar culture medium, R2A agar culture medium, YPD solid culture medium and BSM solid culture medium; And its improved, deformed and optimized types.

The above types of semi-solid fermentation include but are not limited to: batch fermentation. The culture medium used in the semi-solid fermentation method includes but is not limited to: LB culture medium, YEB culture medium, Sabiol agar culture medium, R2A agar culture medium, YPD culture medium; And its improved, deformed and optimized types.

The application method recommended by the present disclosure for patients with sleep behavior disorder during rapid eye movement sleep period is as follows: the probiotic capsule of the present disclosure is 250mg per capsule, in which the number of live bacteria in the capsule meets the requirements of the present disclosure. The weight of the probiotic freeze-dried powder contained in the capsule is related to the preparation method of the dry powder, and a slight difference is also feasible. Usage: Take orally, two capsules each time, three times a day.

The recommended use method of the present disclosure for patients with Parkinson's disease is: the probiotic capsule of the present disclosure is 250mg per capsule, in which the number of live bacteria in the capsule meets the requirements of the present disclosure. The weight of the probiotic freeze-dried powder contained in the capsule is related to the preparation method, and a slight difference is also feasible. Usage: Take orally, two capsules each time, three times a day.

In the present disclosure, the probiotic composition and the probiotic preparation ingredients prepared from the probiotic composition work together, which is proved by clinical trials to be effective in treating and improving the RBD symptoms of patients, with good persistence and stability of RBD symptoms. The present disclosure also has therapeutic effect on RBD symptoms of PD patients, and can effectively improve the exercise symptoms of PD patients, solving the problem that existing drugs cannot improve the treatment of exercise symptoms and non-exercise symptoms of PD patients at the same time. The probiotic preparation provided by the present disclosure has no side effects, will not produce drug dependence and resistance, and can effectively reduce the dosage of levodopa in PD patients, prevent patients from developing resistance to levodopa prematurely, and has a better protective effect on patients. The present disclosure has good clinical application prospects and market prospects.

### BRIEF DESCRIPTION OF THE DRAWINGS

FIG. 1 is graph showing the changes of RBD-HK scores in three groups of RBD+PD patients.
FIG. 2 is graph showing the changes of UPDRS-III scores from baseline in three groups of RBD+PD patients after 12 and 24 weeks of treatment.
FIG. 3 is graph showing the comparison of the improvement rate of motor symptoms in three groups of RBD+PD patients after 12 weeks of treatment.
FIG. 4 is graph showing the comparison of the improvement rate of motor symptoms in three groups of RBD+PD patients after 24 weeks of treatment.
FIG. 5 is graph showing the variation trend of levodopa equivalent dose in three groups of RBD+PD patients.
FIG. 6 is graph showing the comparison of RBD-HK scores before and after iRBD treatment.

### DETAILED DESCRIPTION

The inventors have confirmed through clinical trials that the combination of *Bacillus licheniformis, Bifidobacterium longum, Lactobacillus acidophilus,* and *Enterococcus faecalis* has a better efficacy on the RBD symptoms in patients with idiopathic RBD and patients with Parkinson's disease, with better persistence and stability of efficacy, and has a protective effect on the disease progression in the patients with Parkinson's disease. The present disclosure has no specific requirements on the viable count of the above four strains, and only needs to meet the local production standards of the probiotic products and can be colonized in the intestinal tract of patients. The viable count of each strain described in the specific embodiments of the present disclosure is a recommended and better solution. The specific embodiments are as follows.

The lyophilized probiotic powder as raw materials used in the examples of the present disclosure are commercially available. Specific purchase sources: Jingyue Biotechnology Co., Ltd., Shanenkang Biotechnology (Suzhou) Co., Ltd., Beijing Beina Chuanglian Biotechnology Research Institute.

In addition to being purchased commercially, the lyophilized probiotic powder can be prepared by the related art, and the specific preparation method can include the following steps:
(1) The seed strain in the seed medium is inoculated to obtain the inoculated product.
(2) The inoculated product in step (1) is placed in a fermentation medium for fermentation, and the pH remains unchanged during the fermentation to obtain a fermentation product.
(3) The fermentation product obtained in step (2) and a protective agent are lyophilized to obtain a lyophilized bacterial powder.

The above strains are *Bacillus licheniformis, Bifidobacterium longum, Lactobacillus acidophilus,* or *Enterococcus faecalis.*

The above protective agent is a well-known ingredient in the art and can be selected from skimmed milk powder, allolactose, Vc-Na, starch, sodium glutamate, etc.

The above culture for seed strain is a conventional technique, which can be found in "Studies on Formation of Lactic Acid and Growth of Bacillus Licheniformis by Biotin Variation" (Chinese Journal of Biotechnology, January 1998), "Optimization of Bifidobacterium Longum Enrichment Medium" (Science and Technology of Food Industry, No. 04, 2004), "Optimization of Lactobacillus Acidophilus Enrichment Medium" (Science and Technology of Food Industry, No. 06, 2002), and "Study on Health Care Yogurt Starter for Lactobacillus Acidophilus and Streptococcus Faecalis" (Journal of Anhui Agricultural Technology Normal University, No. 01, 2000).

The above fermentation culture is a conventional technique, and reference can be made to the related art: "Optimization of Bacillus Licheniformis 10236 Fermentation Conditions" (Journal of Hebei Agricultural Science, No. 05, 2019), "Optimization of Liquid Fermentation Conditions and Determination of Growth Curve for Bifidobacterium Longum" (Preparation and Technology, Vol. 9, No. 31, November 2012"), "Development of Lyoprotectant for Lactobacillus Acidophilus and Derived Direct-Vat-Set Composite Culture" (Modern Food Science Technology, 2019, 35(09):248-257.), and "Optimization of Encapsulated Fermentation Medium for Enterococcus Faecalis by Response Surface Method" (Science and Technology of Food Industry, No. 21, 2019)

The method for preparing the lyophilized bacterial powder is a conventional technology, and reference may be made to well-known technologies such as CN110367543A, CN110720638A, and CN102356912B.

The probiotic composition, preparation method and colony standard of the present disclosure all conform to Chinese Pharmacopoeia, Edition 2010, Part III and Chinese Biological Products Regulation, Edition 2000.

The CFU/g in the present disclosure means the number of colonies contained in per g of the tested sample, and CFU is the colony forming unit. The CFU/g in the following examples generally refers to "equal to" or "not less than". In actual use, in order to ensure the viable count within the effective range during the shelf life and the survival rate of strains in the intestinal tract of patients after administration, the viable count in the product is generally "not less than".

Probiotic capsules are prepared in the following examples of the present disclosure, and the equipment as used includes a three-dimensional motion mixer (SBH series, Jiangsu Guibao Group Co., Ltd.) and a capsule filling machine (Beijing Guowei Hejiao Medical Equipment Co., Ltd.). The probiotic composition of the present disclosure can also be prepared as powders, granules or tablets by those skilled in the art, if needed. The adjuvants used in each dosage form can be determined and prepared according to common knowledge in the art. The dosage forms, adjuvants and preparation methods do not affect the final technical effect of the present disclosure, and the type of adjuvants, the amount of adjuvants, the proportional relationship between adjuvants and the preparation method are not intended to limit the protection scope of the present disclosure. Of course, according to the actual situation, it is possible to choose not to prepare by oneself. A triple viable formulation available in the art that meets the viable count described in the present disclosure, such as *Bifidobacterium longum, Lactobacillus acidophilus* and *Enterococcus faecalis* triple viable formulation, and a *Bacillus licheniformis* viable capsule that meets the viable count described in the present disclosure can be chosen, and the combination of the two can also achieve the technical effect of the present disclosure.

### Example 1 Preparation of the probiotic formulation in the present disclosure

(1) Dried and lyophilized bacterial powders of *Bacillus licheniformis, Bifidobacterium longum, Lactobacillus acidophilus,* and *Enterococcus faecalis* and adjuvants were mixed to obtain a homogeneous mixture; and the adjuvants were pre-gelatinized starch and lactose in this example.
(2) The mixture obtained in step (1) were dispensed into capsules to obtain capsule formulation, in which the capsules of the capsule formulation were enteric capsules and each capsule contained 250 mg of mixed bacterial powder.

Each capsule contained *Bacillus licheniformis* of not less than 10×10⁸ CFU/g, *Bifidobacterium longum* of not less than 4.0×10⁸ CFU/g, *Lactobacillus acidophilus* of not less than 1.0×10⁸ CFU/g, and *Enterococcus faecalis* of not less than 0.5×10⁸ CFU/g.

After satisfying the above viable counts, the remainder was the adjuvant and the mass ratio of pre-gelatinized starch to lactose was 1: 1.

### Example 2 Preparation of the probiotic formulation in the present disclosure

(1) Dried and lyophilized bacterial powders of *Bacillus licheniformis, Bifidobacterium longum, Lactobacillus acidophilus,* and *Enterococcus faecalis* and adjuvants were mixed to obtain a homogeneous mixture; and the adjuvants were pre-gelatinized starch and lactose in this example.
(2) The mixture obtained in step (1) were dispensed into capsules to obtain capsule formulation, in which the capsules of the capsule formulation were enteric capsules and each capsule contained 250 mg of mixed bacterial powder.

Each capsule contained *Bacillus licheniformis* of not less than 10×10⁹ CFU/g, *Bifidobacterium longum* of not less than 4.0×10⁹ CFU/g, *Lactobacillus acidophilus* of not less than 1.0×10⁹ CFU/g, and *Enterococcus faecalis* of not less than 0.5×10⁹ CFU/g.

After satisfying the above viable counts, the remainder was the adjuvant and the mass ratio of pre-gelatinized starch to lactose was 1: 1.

### Example 3 Preparation of the probiotic formulation in the present disclosure

(1) Dried and lyophilized bacterial powders of *Bacillus licheniformis, Bifidobacterium longum, Lactobacillus acidophilus,* and *Enterococcus faecalis* and adjuvants were mixed to obtain a homogeneous mixture; and the adjuvants were pre-gelatinized starch and lactose in this example.
(2) The mixture obtained in step (1) were dispensed into capsules to obtain capsule formulation, in which the capsules of the capsule formulation were enteric capsules and each capsule contained 250 mg of mixed bacterial powder.

Each capsule contained *Bacillus licheniformis* of not less than 10×10¹⁰ CFU/g, *Bifidobacterium longum* of not less than 4.0×10¹⁰ CFU/g, *Lactobacillus acidophilus* of not less than 1.0×10¹⁰ CFU/g, and *Enterococcus faecalis* of not less than 0.5×10¹⁰ CFU/g.

After satisfying the above viable counts, the remainder was the adjuvant and the mass ratio of pre-gelatinized starch to lactose was 1: 1.

### Example 4 Preparation of the probiotic formulation in the present disclosure

(1) Dried and lyophilized bacterial powders of *Bacillus licheniformis, Bifidobacterium longum, Lactobacillus acidophilus,* and *Enterococcus faecalis* and adjuvants were mixed to obtain a homogeneous mixture; and the adjuvants were pre-gelatinized starch and lactose in this example.
(2) The mixture obtained in step (1) were dispensed into capsules to obtain capsule formulation, in which the capsules of the capsule formulation were enteric capsules and each capsule contained 250 mg of mixed bacterial powder.

Each capsule contained *Bacillus licheniformis* of not less than 1×10⁷ CFU/g, *Bifidobacterium longum* of not less than 1×10⁷ CFU/g, *Lactobacillus acidophilus* of not less than 1×10⁷ CFU/g and *Enterococcus faecalis* of not less than 1×10⁷ CFU/g.

After satisfying the above viable counts, the remainder was the adjuvant and the mass ratio of pre-gelatinized starch to lactose was 1: 1.

### Efficacy trial 1 of the present disclosure - Parkinson's disease cohort study

### I. Materials and methods:

### a) Subjects

1. Inclusion criteria:
   To be eligible for this study, subjects must meet all of the following inclusion criteria:
   1) Those who were aged 40 to 80 years old, male or female;
   2) Patients with primary Parkinson's disease, were in line with the MDS clinical diagnostic criteria for Parkinson's disease (2015); modified Hoehn and Yahr grading of Parkinson's disease ≤ 3; no dementia, Mini-mental State Examination (*MMSE*) score > 24;
   3) The motor symptoms and RBD symptoms of Parkinson's disease were stable for 1 month before enrollment, and all administration was not adjusted for 1 month before enrollment; no clonazepam or melatonin or other benzodiazepines were administrated for 1 month before enrollment;
   4) No intestinal probiotics or prebiotics (including lactulose) or antibiotics were administrated for 2 months before enrollment; if administrated, a 2-month washout period should be performed; and
   5) Those who understood and agreed to follow the research protocol, and agreed to enroll and sign the informed consent form.
2. Exclusion criteria:
   Subjects who meet any of the following criteria are not eligible for this study:
   1) Those who had Parkinsonism-plus syndrome and secondary Parkinson's syndrome, such as multiple system atrophy, progressive supranuclear palsy, corticobasal degeneration, dementia with Lewy body, vascular Parkinson's syndrome, postencephalitic Parkinsonism, or any other non-primary Parkinson's disease;
   2) Any probiotics or prebiotics (including lactulose) or antibiotics were administrated within 2 months before enrollment;
   3) Those who had the following diseases: such as Alzheimer's disease, malignant tumor, myelopathy, epilepsy, obstructive sleep apnea syndrome, autonomic nervous disease (urinary retention, urinary incontinence or orthostatic hypotension, blood pressure drop of more than 30/15 mmHg after standing for 5 minutes), etc.; new-onset cerebrovascular disease or severe sequelae of cerebrovascular disease affecting the assessors within 3 months;
   4) Those who had history of gastrointestinal tumor, inflammatory bowel disease, and other acute and chronic inflammations of the gastrointestinal tract within 3 months (including acute exacerbation of cholecystitis);
   5) Those who had severe cardiovascular disease (such as American Heart Association function class III-IV congestive heart failure, history of myocardial infarction within 6 months, etc.);
   6) Those who had severe liver and kidney dysfunction, glutamic-pyruvic transaminase, glutamic oxalacetic transaminase, and total bilirubin of higher than 1.5 times the upper limit of normal; serum creatinine of 1.5 times higher than the upper limit of normal;
   7) Those who were pregnant and lactating women or women of reproductive age between 40 and 60 years old with positive HCG;
   8) Those who were known to be allergic to the test drug or related products;
   9) Those who had a history of drug abuse or alcohol dependence;
   10) Those who participated in other clinical trials within 3 months before enrollment; and
   11) Those who refused to be enrolled and cannot cooperate with the researcher; and those who were judged by the researcher to be unsuitable for enrollment.
3. Randomization scheme: this study was a prospective open cohort study. According to whether the patients had RBD, the patients were further stratified into RBD+PD patients and RBD-PD patients, and the patients in each stratum were randomly divided into test group 1, test group 2 and control group.
   RBD+ criteria (Stiasny-Kolster K, Mayer G, Schafer S, et al. The REM sleep behavior disorder screening questionnaire - a new diagnostic instrument [J]. Mov Disord, 2007, 22(16): 2386-2393. Shen SS, Shen Y, Xiong KP, Chen J, Mao CJ, Huang JY, Li J, Han F, Liu CF, Validation study of REM sleep behavior disorder questionnaire-Hong Kong (RBDQ-HK) in east China. Sleep Med. 2014 Aug; 15(8):952-8. doi: 10.1016/j.sleep.2014.03.020.): For Rapid eye movement sleep behavior disorder screening questionnaire (RBDSQ), RBDSQ> 5, for Rapid eye movement sleep behavior disorder questionnaire Hong Kong (RBD-HK for short), RBD-HK score ≥ 17, and RBD is diagnosed by polysomnography; and those who did not meet the above criteria were assigned as RBD-.
4. Drop out, discontinuation and withdrawal scheme: patients who cannot continue to participate in this study, or patients who cannot be contacted during multiple follow-up are considered as drop out; those who developed any inflammation required to be treated with antibiotics during the observation period will have unaccepted scale scores during the use period and are considered as discontinuation; and those who developed acute cardiovascular and cerebrovascular accidents or other serious adverse events are considered as withdrawal.

### II. Test method:

a) Intervention samples: Test group 1 (intervention group): the probiotic formulation was obtained in Example 1 of the present disclosure. Test group 2 (intervention group): each capsule contained a probiotic capsule containing 1.0×10⁷ CFU of *Bifidobacterium longum,* 1.0×10⁷ CFU of *Lactobacillus acidophilus,* and 1.0×10⁷ CFU of *Enterococcus faecalis* (purchased from Shanghai Sine Pharmaceutical Laboratories Co., Ltd., National Medicine Permission Number S10950032). Control group: only pre-enrollment therapy thereof was maintained, and no probiotics or prebiotics were added.
b) Test method:
   On the basis of conventional treatment of PD, test group 1 was given the formulation of Example 1, 2 capsules each time, three times a day. On the basis of conventional treatment of PD, test group 2 was given the formulation of test group 2 according to the instruction, 4 capsules each time, twice a day. The blank control group was only given conventional PD drugs.
   The daily dose of all anti-PD drugs was converted into levodopa equivalent dose according to the following conversion formula: 100 mg levodopa = 100 mg piribedil = 1 mg pramipexole = 5 mg ropinirole = 10 mg selegiline = 100 mg amantadine = 133 mg carbidopa = 1 mg rasagiline. The dose of entacapone was calculated as levodopa equivalent dose with a 33% potentiation.
   After enrollment, RBD+PD patients were not given clonazepam or other benzodiazepines or melatonin or the like for sleep intervention until the end of the observation period.
c) Evaluation: All patients were collected for electrocardiogram, blood routine, liver and kidney function, and electrolyte testing at the time of enrollment and week 12. Rapid eye movement sleep behavior disorder screening questionnaire (RBDSQ) was used for screening, and rapid eye movement sleep behavior disorder questionnaire Hong Kong (RBD-HK) was used to assess RBD and the severity thereof; and Unified Parkinson's Disease Rating Scale (UPDRS for short) Part III UPDRS-III was used as an indicator for evaluating the severity of motor symptoms. The difference of the score at week 12 or week 24 minus the score at week 0 was used for statistics. The difference > 3 was regarded as aggravation, the difference <-3 was regarded as improvement, and the difference between -3 and 3 was regarded as stability. The UPDRS-III score and RBD-HK score were completed at week 0, week 12, and week 24 after enrollment, respectively, and the names and doses of patients' conventional anti-PD drugs were recorded.
d) Data statistics: SPSS26.0 was used for statistical analysis of the data. Measurement data were expressed as mean±standard deviation (x±s), and enumeration data were expressed as percentage. The data at week 12 was used as the main statistical indicator. Paired t-test was used for intra-group comparison of measurement data; analysis of variance was used for comparison among three groups; analysis of variance was used for comparison among three groups at different time points; and chi-square test or Fisher's exact test was used for enumeration data. P < 0.05 meant that the difference was statistically significant.

### III. Test results and analysis

### General patient information

This study was approved by Ethics Committee of Beijing Friendship Hospital, Capital Medical University.

Patients attending the neurology outpatient clinic and ward at Beijing Friendship Hospital, Capital Medical University from May 2018 to December 2019 were collected, all of whom signed an informed consent form.

A total of 62 patients with primary Parkinson's disease who met the inclusion criteria were collected, 20 in test group 1, 20 in test group 2, and 22 in control group with 2 drop-out cases. Finally, 60 patients were enrolled. 48 cases of RBD+ and 12 cases of RBD- were randomly assigned to three groups with 16 cases of RBD+PD patients and 4 cases of RBD-PD patients in each group. There were 35 males with a median age of 66 years, and there was no significant differences in age (P < 0.05, analysis of variance) and gender (P < 0.05, chi-square test) distribution between the three groups. There was no obvious abnormality in electrocardiogram, blood routine, liver and kidney function, electrolytes, etc. at the time of enrollment and week 12, and no patients withdrew from observation in advance due to the above reasons. No patients withdrew from observation due to serious adverse events. The H-Y grading was 2.10±0.68, 2.15±0.65, and 1.75±0.60 for the three groups of patients, respectively, with no statistically significant difference (P = 0.110 > 0.05, analysis of variance).

### a) Comparison of RBD effects

### 1. 12-week follow-up results

As shown in Table 1, at the time of enrollment, RBD-HK scores of 16 RBD+PD patients in all groups were 38.75±10.89 (test group 1), 33.19±12.50 (test group 2), and 30.56±12.79 (control group), respectively, with no statistically significant difference among three groups (P=0.178>0.05, analysis of variance). After 12 weeks of treatment, compared to week 0 in this group, the differences in RBD-HK scores were statistically significant in both test group 1 and test group 2 (p < 0.001 (test group 1), p < 0.05 (test group 2)), while there was no significant difference in the scores of the control group before and after enrollment (p > 0.05). For the comparison of the changes in RBD-HK scores from baseline among three groups, the difference between the two groups was statistically significant (p < 0.05); and the differences of changes in RBD-HK scores from baseline between test group 1 and control group (p < 0.001), test group 2 and control group (p = 0.025), and test group 2 and test group 1 (p = 0.035) were statistically significant (p < 0.05. analysis of variance).

**Table 1 RBD-HK scores of patients with RBD + PD in three groups at week 12**

| Group | Week 0 | Week 12 | Change from baseline |
|---|---|---|---|
| Test group 1 (n = 16) | 38.75±10.89 | 29.13±12.46*** | -9.63±6.1^{###} |
| Test group 2 (n = 16) | 33.19±12.50 | 29±12.90* | -4.19±6.31^{#} |
| Control group (n = 16) | 30.56±12.79 | 32.06±11.97 | 1.5±5.03 |

| | | | |
|---|---|---|---|
| *P < 0.05, **P < 0.01, and ***P < 0.001, compared to this group before treatment; and # P < 0.05, ## P < 0.01, and ### P < 0.001, compared to the control group after treatment. | | | |

### 2. 24-week follow-up RBD results:

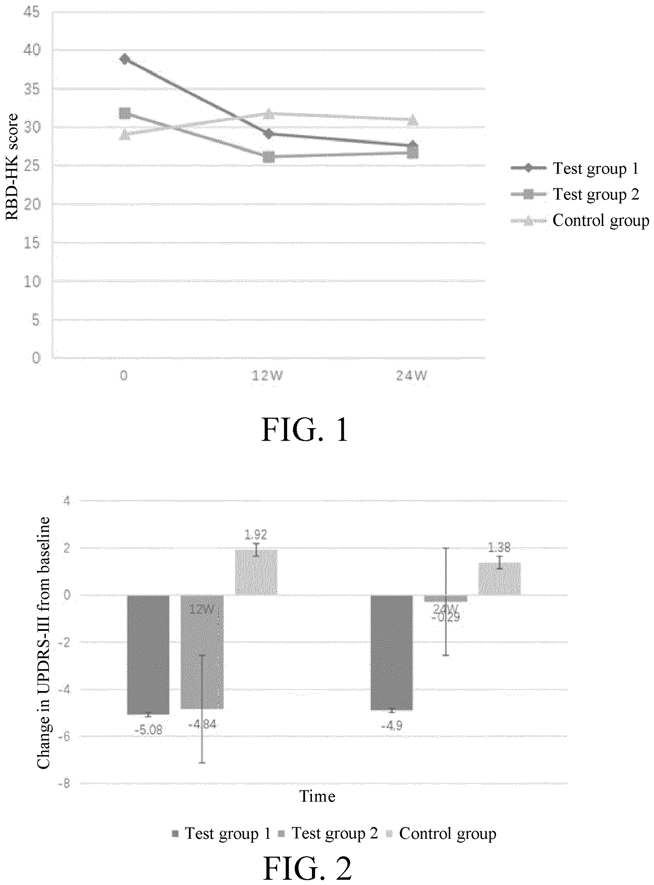

See Table 2 and FIG. 1 for details, 14, 11, and 14 cases in the three groups completed 24-week follow-up, respectively (reasons for drop out: some patients failed to meet the period for 24-week follow-up, and a small number of patients could not be followed up because of the current 2019-2020 COVID-19 pandemic). For the comparison of the changes in RBD-HK scores from baseline, there was a statistically significant difference among three groups (p < 0.05); and the differences of changes in RBD-HK scores from baseline between test group 1 and control group (p < 0.001), test group 2 and control group (p = 0.021), and test group 1 and test group 2 (p = 0.048) were statistically significant (p < 0.05. analysis of variance).

**Table 2 RBD-HK scores of patients with RBD + PD in three groups at week 24**

| Group | Week 0 | Week 12 | Change from baseline at week 12 | Week 24 | Change from baseline at week 24 |
|---|---|---|---|---|---|
| Test group 1 (n = 14) | 38.86±10.87 | 29.14±12.23*** | -9.71±6.20^{###} | 27.57±11.29*** | -11.29±7.44^{###} |
| Test group 2 (n = 11) | 31.82±9.91 | 26.18±10.46* | -5.64±5.77 | 26.73±10.20* | -5.09±3.83 |
| Control group (n = 14) | 29.07±11.71 | 31.79±11.62* | 2.71±3.90 | 31±13.24* | 1.93±5.99 |

| | | | | | |
|---|---|---|---|---|---|
| *P < 0.05, **P < 0.01, and ***P < 0.001, compared to this group before treatment; and # P < 0.05, ## P < 0.01, and ### P < 0.001, compared to the control group at the same period. | | | | | |

**In conclusion:** the results suggest that there was a sustained reduction in RBD-HK score in test group 1 (the present disclosure group) over time, i.e., there was a sustained improvement in the patient's RBD condition; for test group 2 (tri-bacteria group), the patients' RBD condition improved at week 12 of treatment compared with that at the time of enrollment, but there was no further improvement after continued treatment; and the RBD-HK scores in the control group were not statistically different at weeks 0, 12, and 24, suggesting that anti-Parkinson's disease motor symptoms drugs had no significant effect on the patients' RBD condition. Further as shown in Figure 1, the RBD-HK score of test group 1 continued to decrease with a significant and stable trend of decrease. Although there was a decrease in test group 2, there was an obvious upward trend in the later period. Although both groups had certain efficacy, the persistence and stability of the efficacy of test group 1 was more excellent than that of test group 2. To sum up, it is suggested that for the RBD symptoms of PD patients, the therapeutic effect of test group 2 was better than that of the control group, and the therapeutic effect of test group 1 of the present disclosure was better than that of test group 2 and much better than that of the control group; and there was a continuous improvement in the efficacy provided by the present disclosure with the extension of treatment time.

### b) Movement symptom data statistics

1. Comparison of UPDRS-III scores: Note: since this scale required patients to come to the hospital for a face-to-face assessment by physicians, some patients missed the assessment time point as they failed to arrive at the hospital within the specified time, and since some patients did not meet the period for the 24-week follow-up, the number of patients that were followed up was partially reduced.

Based on a paired t-test, 19, 16, and 18 patients in the three groups completed the 12-week follow-up of UPDRS scale, respectively, and there was no statistically significant difference among the baseline UPDRS III scores of the three groups (P=0.05, analysis of variance). For intra-group comparison of UPDRS-III scores at week 12 of treatment with week 0, there were statistically significant differences in both test group 1 (P = 0.001) and test group 2 (P = 0.029), while there was no statistically significant difference in the control group (P = 0.111).

See Table 3 for comparison of the change in UPDRS III scores after 12 weeks of treatment and scores at week 0, the changes for the three groups were -5.08±5.82 (test group 1), - 4.84±8.01 (test group 2), and 1.92±4.84 (control group), respectively, and the difference was statistically significant (p < 0.001). The difference of change in UPDRS III scores between test group 1 and control group (p=0.004), and test group 2 and control group (p=0.009) was statistically significant (P<0.01); and there was no statistically significant difference in the change in UPDRS III score between test group 2 and test group 1 (p=1.000>0.05) (variance analysis). However, the change in scores of test group 1 was better improved than that of test group 2 after 12 weeks of treatment.

**Table 3 UPDRS III score of PD patients in the three groups at week 12 of treatment**

| Group | Week 0 | Week 12 | Change from baseline |
|---|---|---|---|
| Test group 1 (n = 19) | 21.66±8.70 | 16.58±8.93** | -5.08±5.82^{##} |
| Test group 2 (n = 16) | 21.78±9.45 | 16.94±7.48* | -4.84±8.01 ^{##} |
| Control group (n = 18) | 15.08±8.27 | 17±8.70 | 1.92±4.84 |

| | | | |
|---|---|---|---|
| *P < 0.05, **P < 0.01, and ***P < 0.001, compared to this group before treatment; and # P < 0.05, ## P < 0.01, and ### P < 0.001, compared to the control group after treatment. | | | |

As shown in Table 4 and FIG. 2, the number of patients who completed the 24-week follow-up of UPDRS score was 15, 7, and 8 in each group, respectively. Based on the statistics on these patients, it was found that for intra-group comparison of UPDRS-III scores at week 24 of treatment with week 0, there was a statistically significant difference in test group 1 (P = 0.020), and no statistically significant difference in both test group 2 (P = 0.907) and control group (P = 0.546). For the change in UPDRS-III scores at week 24 from baseline, there was no statistically significant difference between the three groups (P = 0.09 > 0.05). For the comparison of the change in UPDRS-III scores, there was no statistically significant difference between test group 1 and the control group (p = 0.126), test group 2 and the control group (p = 1.000), and test group 1 and test group 2 (p = 0.435) (p > 0.05) (variance analysis). However, the trend of improvement was more pronounced in test group 1 (-4.9±7.20) than in test group 2 (-0.29±6.18) and control group (1.38±6.13) for the change in UPDRS-III scores at week 24 of treatment.

**Table 4 UPDRS III scores of PD patients in the three groups at week 24**

| Group | Week 0 | Week 24 | Change from baseline |
|---|---|---|---|
| Test group 1 (n = 15) | 19.73±8.06 | 14.83±9.60* | -4.9±7.20 |
| Test group 2 (n = 7) | 18.36±6.87 | 18.07±7.76 | -0.29±6.18 |
| Control group (n = 8) | 16.5±9.50 | 17.88±6.56 | 1.38±6.13 |

| | | | |
|---|---|---|---|
| *P < 0.05, **P < 0.01, and ***P < 0.001, compared to this group before treatment; and # P < 0.05, ## P < 0.01, and ### P < 0.001, compared to the control group after treatment. | | | |

### 2. Improvement rate statistics:

According to the literature, a difference between baseline UPDRS-III score after treatment and that at the time of enrollment of ≤ | ±3 | was defined as stable disease, a negative score of >3 was defined as improvement, and a positive score of >3 was defined as aggravation. Then, after 12 weeks of treatment, there was a statistically significant difference in the improvement rate among the three groups (P = 0.008 < 0.01). See Table 5 for comparison of the efficacy at week 12 of treatment, there was a statistically significant difference (p < 0.05) in the improvement rate between test group 1 and control group (p = 0.004) and test group 2 and control group (p = 0.013), and there was no statistically significant difference (p > 0.05, variance analysis) in the improvement rate between test group 1 and test group 2 (p = 0.691).

**Table 5 Comparison of the therapeutic effects in the three treatment regimens at week 12**

| Group | Therapeutic Effects (UPDRS) | | | Improvement rate |
|---|---|---|---|---|
| | Improvement | Stable disease | Aggravation | |
| Test group 1 (n = 19) | 10 | 8 | 1 | 52.6%^{##} |
| Test group 2 (n = 16) | 9 | 5 | 2 | 56.3%^{#} |
| Control group (n = 18) | 2 | 8 | 8 | 11.1% |

| | | | | |
|---|---|---|---|---|
| *P < 0.05, **P < 0.01, and ***P < 0.001, compared to this group before treatment; and # P < 0.05, ## P < 0.01, and ### P < 0.001, compared to the control group in the same period after treatment. | | | | |

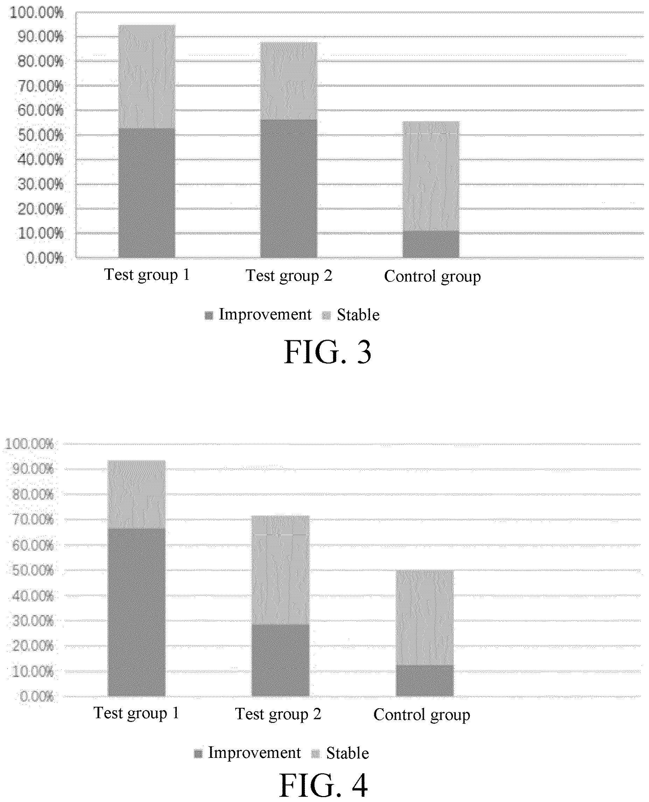

At week 24 of treatment, there was a statistically significant difference (p < 0.05) in the improvement rate between test group 1 and control group (p = 0.015), and there was no statistically significant difference (p > 0.05, analysis of variance) in the improvement rate between test group 1 and test group 2 (p = 0.198) and test group 2 and control group (p = 0.814), and no statistically significant difference in the improvement rate among the three groups (p = 0.062 > 0.05, analysis of variance). However, the improvement rate of 66.7% in test group 1 was significantly higher than that of 28.6% in test group 2. See Table 6 and FIG. 3.

**Table 6 Comparison of the therapeutic effects in the three treatment regimens at week 24**

| Group | Treatment Effectiveness (UPDRS-III) | | | Improvement rate |
|---|---|---|---|---|
| | Improvement | Stable disease | Aggravation | |
| Test group 1 (n = 15) | 10 | 4 | 1 | 66.7%^{#} |
| Test group 2 (n = 7) | 2 | 3 | 2 | 28.6% |
| Control group (n= 8) | 1 | 3 | 4 | 12.5% |

| | | | | |
|---|---|---|---|---|
| *P < 0.05, **P < 0.01, and ***P < 0.001, compared to this group before treatment; and # P < 0.05, ## P < 0.01, and ### P < 0.001, compared to the control group in the same period after treatment. | | | | |

Referring to Table 6 and FIG. 4, the above results suggested that: after administrating probiotic treatment to the patients, both test group 1 and test group 2 significantly improved patients' motor symptoms, and test group 1 had a stronger improvement effect; and with the extension of treatment time, the treatment of motor symptoms was consistently effective in test group 1, the proportion of patients with improved symptoms gradually increased from 52.6% at week 12 to 66.7% at week 24, while the improvement rate in test group 2 (tri-bacteria group) decreased from 56.3% at week 12 to 28.6% at week 24, and the improvement rate in the control group was basically stable at 11.1% to 12.5%. The improvement rate at week 24 was significantly higher in test group 1 than in control group and test group 2. It showed that test group 1 of the present disclosure has a stronger tendency to improve the therapeutic effect of motor symptoms than test group 2, and the persistence and stability of the improvement trend were better in test group 1 than that in test group 2.

In conclusion: after administrating probiotic treatment to the patients, both the test group 1 of the present disclosure and the test group 2 improved the motor symptoms of the patients; compared with test group 2, the present disclosure was better than test group 2 in terms of UPDRS-III score and overall improvement rate; and with the extension of treatment time, the effect on improving motor symptoms gradually increased, and the overall improvement rate gradually increased. At week 24, 66.7% of patients showed significant improvement in motor symptoms, which was much higher than in test group 2 and control group. More importantly, the present disclosure showed a stronger, and more consistent and stable trend of improvement in motor symptoms than test group 2.

### c) Comparison of levodopa equivalent dose

The statistical sample of patients in this group was the same as the statistical sample of patients with motor symptoms.

There was no significant difference in levodopa equivalent dose statistics among the three groups at the time of enrollment (P = 0.227). For the comparison of change in levodopa equivalent dose at week 12 of treatment from baseline, there was a statistically significant difference among the three groups (P = 0.004, <0.05). Both test group 1 and test group 2 showed a reduction in levodopa equivalent dose/day at week 12 compared to that at the time of enrollment, with a reduction of -22.37±45.56 mg/d and -8.59±42.50 mg/d, respectively. The levodopa equivalent dose reduction was statistically significant in test group 1 compared to control group (p = 0.005) (p < 0.05), and there was no statistically significant difference (p > 0.05) in the change of levodopa equivalent dose between test group 2 and control group (p = 0.051), and test group 1 and test group 2 (p = 1.000) (analysis of variance). See Table 7.

**Table 7 Comparison of levodopa equivalent dose of PD patients in three groups at week 12**

| | Levodopa equivalent dose (mg/d) | | |
|---|---|---|---|
| Group | Week 0 | Week 12 | Change from baseline |
| Test group 1 (n = 19) | 329.61±196.57 | 307.24±182.87* | -22.37±45.56^{##} |
| Test group 2 (n = 16) | 437.5±283.39 | 428.91±262.04 | -8.59±42.50 |
| Control group (n = 18) | 311.11±171.03 | 348.61±192.84* | 37.5±69.79 |

| | | | |
|---|---|---|---|
| *P < 0.05, **P < 0.01, and ***P < 0.001, compared to before treatment; and # P < 0.05, ## P < 0.01, and ### P < 0.001, compared to the control group after treatment. | | | |

There was no statistically significant difference in the change in levodopa equivalent dose at week 24 of treatment among the three groups (P = 0.22 > 0.05). There was no statistically significant difference (p > 0.05) in the change in levodopa equivalent dose between test group 1 and control group (p = 0.331), test group 2 and control group (p = 0.491), and test group 1 and test group 2 (p = 1.000) (analysis of variance). See Table 8.

**Table 8 Levodopa equivalent dose of PD patients in three groups at week 24**

| roup | Levodopa equivalent dose (mg/d) | | | | |
|---|---|---|---|---|---|
| | **Week 0** | **Week 12** | **Change baseline at week 12** | **Week 24** | **from Change from baseline at week 24** |
| Test group 1 (n = 15) | 313.33±197.46 | 286.67±176.98 | -26.67±48.71 | 285±165.53 | -28.33±78.41 |
| Test group 2 (n = 7) | 442.86±326.70 | 428.57±310.37 | -14.29±22.59 | 414.29±320.40 | -28.57±39.34 |
| Control group (n = 8) | 359.38±135.75 | 378.13±161.75 | 18.75±42.85 | 378.13±161.75 | 18.75±45.81 |

| | | | | | |
|---|---|---|---|---|---|
| *P < 0.05, **P < 0.01, and ***P < 0.001, compared to before treatment; and # P < 0.05, ## P < 0.01, and ### P < 0.001, compared to the control group after treatment. | | | | | |

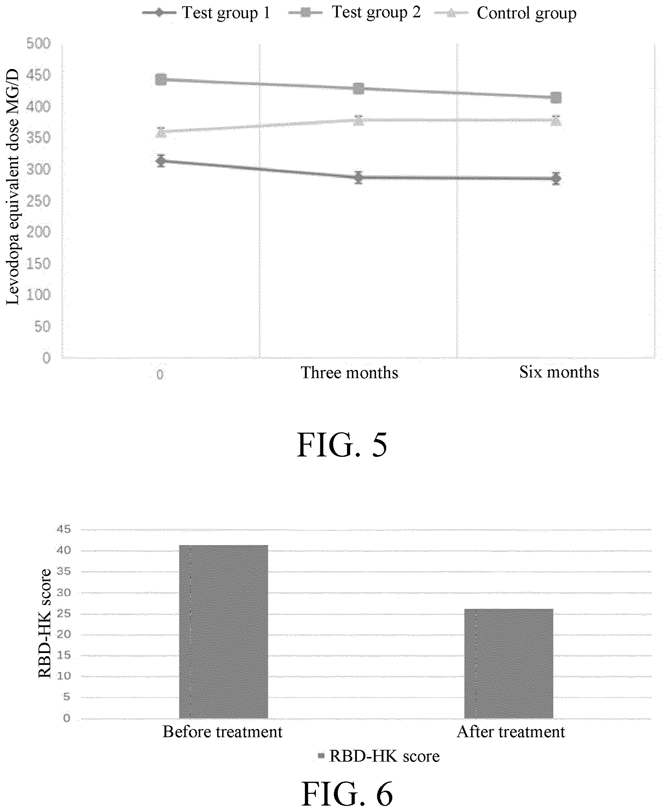

Patients who completed both the UPDRS III scores at weeks 12 and 24 and levodopa equivalent dose recordings were 15 in test group 1, 7 in test group 2, and 8 in control group, and the change in levodopa equivalent dose among the three groups was shown in FIG. 5.

In conclusion: after the treatment of test group 1 of the present disclosure, the patients' RBD severity was improved significantly, and the efficacy was significantly higher than that of test group 2; moreover, the improvement effect gradually increased over time; and along with the improvement of RBD, the patients' motor symptoms were significantly improved, and the improvement rate tended to increase significantly, while the daily levodopa equivalent dose was significantly reduced compared with that at the time of enrollment, suggesting that the improvement of motor symptoms resulted from probiotics rather than the increase of levodopa equivalent dose. There was a trend towards improved efficacy in test group 1 compared to test group 2.

The probiotic formulation prepared in Example 1 was used in the above clinical trial, and the same test conclusion can be achieved via testing the probiotic formulation prepared in Example 4 by the inventors. Theoretically, the technical effect of the present disclosure can be achieved by the probiotic having a viable count of not less than the CFU/g as described. Taking into account the loss rate of live bacteria during storage and transportation of probiotics, a slightly lower viable count than the limit of the present disclosure is also feasible.

### Efficacy trial 2 of the present disclosure - idiopathic RBD (iRBD) Cohort Study

### I. Materials and methods:

### a) Subjects

1. Inclusion criteria:
   To be eligible for this study, subjects must meet all of the following inclusion criteria:
   1) Those who were aged 40 to 85 years old, male or female;
   2) For Rapid eye movement sleep behavior disorder screening questionnaire (RBDSQ), RBDSQ > 5, and for Rapid eye movement sleep behavior disorder questionnaire Hong Kong (RBD-HK for short), RBD-HK score ≥ 17; and
   3) Diagnostic criteria for iRBD: Diagnosis was confirmed by a physician at Department of Neurology & Sleep Disorders Center based on polysomnography (PSG for short), where the patient had a history of dream enactment behavior, and video polysomnography showed increased electromyography activity or abnormal behavior during the rapid eye movement.
2. Exclusion criteria:
   1) Those with a history of serious mental illness or drug abuse; a Hamilton Depression Scale score of ≥ 17, and/or an anxiety scale score of ≥ 14 or being treated with oral antidepressant or antipsychotics; being treated with oral clonazepam or other benzodiazepines and/or melatonin within 4 weeks; and
   2)Those who had other central nervous system diseases, including cerebrovascular disease, brain tumors, inflammation, genetic metabolic diseases, traumatic brain injury, definite degenerative diseases of the central nervous system (e.g. Parkinson's disease, multiple system atrophy, dementia with Lewy body, etc.), and the like.
3. Drop out, discontinuation and withdrawal scheme: Patients who cannot continue to participate in this study, or patients who cannot be contacted during multiple follow-up are considered as drop out; those who developed any inflammation required to be treated with antibiotics during the observation period will have unaccepted scale scores during the use period and are considered as discontinuation; and those who developed acute cardiovascular and cerebrovascular accidents or other serious adverse events are considered as withdrawal.

### b) Test method:

1. Intervention method: The probiotic formulation was obtained in Example 4 of the present disclosure, 2 capsules each time, three times a day, for 12 weeks of continuous intervention.
2. Evaluation: All patients were collected for electrocardiogram, blood routine, liver and kidney function, and electrolyte testing at the time of enrollment and week 12. Rapid eye movement sleep behavior disorder screening questionnaire (RBDSQ) was used for screening, and rapid eye movement sleep behavior disorder questionnaire Hong Kong (RBD-HK) was used to assess RBD and the severity thereof.
3. Data statistics: SPSS26.0 was used for statistical analysis of the data. Measurement data were expressed as mean±standard deviation (x±s), and the data at week 12 was used as the main statistical indicator. Paired t-test was used; and P < 0.05 meant that the difference was statistically significant.

### II. Test results and analysis

This study was approved by Ethics Committee of Beijing Friendship Hospital, Capital Medical University.

Patients attending the neurology outpatient clinic and ward at Beijing Friendship Hospital, Capital Medical University from January 2019 to January 2021 were collected, all of whom signed an informed consent form.

A total of 6 iRBD patients, 5 males and 1 female, with a mean age of 64.7±2.7 years, who met the inclusion criteria and did not meet the exclusion criteria were collected. The RBDSQ scores of all patients were >5. The RBD-HK score before the intervention was 41.50±15.67. After 12 weeks of treatment, the RBD condition improved significantly in 5 patients and remained stable in 1 case, with a RBD-HK score of 26.17±9.99, which was statistically significantly different from that before the intervention (P = 0.038, P < 0.05), as shown in FIG. 6.

### III. In conclusion

After the present disclosure was used for the treatment of idiopathic RBD (iRBD), most of the patients (5/6 cases) had a significantly improved RBD condition compared with that before treatment, and 1 case had a stable onset condition, suggesting that the present disclosure can significantly improve the symptoms of idiopathic RBD. Currently, no related reports have been seen. Idiopathic RBD is a prodromal manifestation of neurodegenerative diseases especially Parkinson's disease. The present disclosure ameliorates the onset of idiopathic RBD and is expected to delay the risk of developing neurodegenerative diseases such as PD or dementia with Lewy body in iRBD patients.

## Claims

1. A probiotic composition for treating rapid eye movement sleep behavior disorder, comprising *Bacillus licheniformis, Bifidobacterium longum, Lactobacillus acidophilus,* and *Enterococcus faecalis* and acceptable adjuvants in the art.

2. The probiotic composition for treating rapid eye movement sleep behavior disorder according to claim 1, wherein
the viable count of *Bacillus licheniformis* in the probiotic composition is not less than 1×10⁷ CFU/g;
the viable count of *Bifidobacterium longum* in the probiotic composition is not less than 1×10⁷ CFU/g;
the viable count of *Lactobacillus acidophilus* in the probiotic composition is not less than 1×10⁷ CFU/g;
the viable count of *Enterococcus faecalis* in the probiotic composition is not less than 1×10⁷ CFU/g.

3. A probiotic composition for treating rapid eye movement sleep behavior disorder, wherein the probiotic composition is made of *Bacillus licheniformis, Bifidobacterium longum,* Lactobacillus acidophilus, Enterococcus faecalis and pharmaceutically acceptable adjuvants, and,
the viable count of *Bacillus licheniformis* in the probiotic composition is not less than 10.0×10⁸ CFU/g;
the viable count of *Bifidobacterium longum* in the probiotic composition is not less than 4.0×10⁸ CFU/g;
the viable count of *Lactobacillus acidophilus* in the probiotic composition is not less than 1.0×10⁸ CFU/g;
the viable count of *Enterococcus faecalis* in the probiotic composition is not less than 0.5×10⁸ CFU/g.

4. The probiotic composition of claim 1, 2 or 3 is used for preparing probiotic preparations for treating rapid eye movement sleep behavior disorder.

5. The probiotic composition of claim 1, 2 or 3 is used for the preparation of drugs, health products and/or food for the treatment of rapid eye movement sleep behavior disorder.

6. A preparation method of a probiotic composition for the treatment of rapid eye movement sleep behavior disorder, wherein:
the preparation method comprises mixing the four bacteria and adjuvants of claim 1, 2 or 3 into freeze-dried powder.

7. Use of the probiotic composition prepared in claim 6 in the preparation of drugs, health products and/or food for the treatment of rapid eye movement sleep behavior disorder and/or exercise symptoms.

8. A probiotic preparation for the treatment of rapid eye movement sleep behavior disorder, wherein:
the probiotic preparation is made from the probiotic composition of claim 1, 2 or 3 and the acceptable adjuvants in the art.

9. The probiotic preparation of claim 8 is used for the preparation of drugs, health products and/or food for the treatment of rapid eye movement sleep behavior disorder.

10. A method for preparing probiotics according to claim 8, which is **characterized in that** it comprises the following steps:
(1) preparing of bacterial powder: respectively prepare the dried bacterial powder of *Bacillus licheniformis, Bifidobacterium longum, Lactobacillus acidophilus* and *Enterococcus faecalis;*
(2) mixing each dried bacterial powder obtained in step (1) with pharmaceutically acceptable adjuvants to prepare the final dosage form.

11. A food composition or supplement for the treatment of rapid eye movement sleep behavior disorder, wherein:
the food composition or supplement is made of *Bacillus licheniformis, Bifidobacterium longum, Lactobacillus acidophilus, Enterococcus faecalis* and acceptable adjuvants in the art.

12. The food composition or supplement according to claim 11, wherein:
the viable count of *Bacillus licheniformis* in the probiotic composition is not less than 1×10⁷ CFU/g;
the viable count of *Bifidobacterium longum* in the probiotic composition is not less than 1×10⁷ CFU/g;
the viable count of *Lactobacillus acidophilus* in the probiotic composition is not less than 1×10⁷ CFU/g;
the viable count of *Enterococcus faecalis* in the probiotic composition is not less than 1×10⁷ CFU/g.

13. Use of the food composition or supplement according to claim 11 or 12 in the preparation of a health product supplement and/or food for the treatment of rapid eye movement sleep behavior disorder.
